Europäisches Patentamt

European Patent Office

Office européen des brevets

(11) Publication number: 0 249 350
A1

# (12) EUROPEAN PATENT APPLICATION

(21) Application number: 87304477.0

(22) Date of filing: 20.05.87

(51) Int. Cl.⁴: **C12N 15/00** , C07H 21/04 , C12N 5/00 , ///(C12N5/00;C12R1:66)

(30) Priority: 06.06.86 US 871532

(43) Date of publication of application:
16.12.87 Bulletin 87/51

(84) Designated Contracting States:
AT BE CH DE ES FR GB GR IT LI LU NL SE

(71) Applicant: PANLABS, INC.
9725 Third Avenue, N.E.
Seattle, WA 98115(US)

(72) Inventor: Finkelstein, David B.
5535 56th Avenue S.
Seattle Washington 98118(US)
Inventor: Leach, Janette
1401 5th Avenue
Seattle Washington 98115(US)
Inventor: McAda, Phyllis C.
9226 15th Avenue NE
Seattle Washington 98115(US)
Inventor: Rambosek, John A.
8923 Ravenna Avenue NE
Seattle Washington 98115(US)
Inventor: Soliday, Charles L.
14235 209 Avenue NE
Woodinville Washington 98072(US)

(74) Representative: Harrison, David Christopher
et al
MEWBURN ELLIS & CO 2/3 Cursitor Street
London EC4A 1BQ(GB)

(54) Filamentous fungal expression systems.

(57) Novel expression constructs for secretion of protein products in filamentous fungal hosts are provided. The hosts are transformed with the constructs, normally under conditions for multiple integration of the constructs into the host genome, where the desired protein products are expressed and secreted from the host to provide for enhanced yields of the product, substantially free of host proteins. Efficient filamentous fungal promoters are employed which may be from the same or different gene from the signal leader sequence.

## FILAMENTOUS FUNGAL EXPRESSION SYSTEMS

The ability to transform a wide variety of cells with functional DNA, resulting in a change in the genotype or phenotype of the host cell has provided substantial opportunities to produce a wide variety of proteins. Numerous mammalian proteins have been reported as having been produced in unicellular microorganisms, such as bacteria and yeast. While bacteria and yeast have many desirable features for the production of heterologous proteins, these organisms also have many deficiencies. One of the problems encountered with many heterologous proteins is the proper processing and folding. Processing can take the form of many different modifications. Included in processing is glycosylation, terminal amino acylation, cleavage of peptide bonds by exopeptidases and endopeptidases, hydroxylation, and the like.

Besides the above processing, various signal leaders may be employed, which may be involved in the transfer of the peptide to the periplasmic space, to the cell membrane, or to the medium. Besides the concerns with processing, there is also the concern with folding and the deformation of inclusion bodies. Frequently, where the protein of interest which is produced is produced in relatively large amounts, inclusion bodies are obtained which only can be difficultly·renatured, resulting in loss of product, loss of activity and difficulties in isolation of the active material free of the denatured protein.

There is, therefore, a continued interest in developing new systems for the production of proteins. In each instance, the system must be concerned with the ability to grow the host under economical conditions, the efficiency with which physiologically active proteins are produced, and the ease with which the products may be isolated from the host or the nutrient medium.

Brief Description of the Relevant Literature

Collins, et al., Cell (1981) 24:443-452 describes the characterization of a novel plasmid found in mitochondria from N. crassa. Case, et al., Proc. Natl. Acad. of Sci. U.S.A. (1979) 76:5259-5263 and Stahl, et al., Proc. Natl. Acad. of Sci. U.S.A. (1982) 79:3641-3645 describe the transformation, utilizing hybrid plasmid DNA, of N. crassa and Podospora, respectively. Kelly and Hynes, EMBO J. (1985) 4:475-479, report transformation of A. niger using the amdS gene of A. nidulans. Paietta and Marzluf, Mol. and Cell. Biol. - (1985) 5:1554-1559 report that bacterial sequences may not be required to transform N. crassa. Nunberg, et al., Mol. and Cell. Biol. (1984) 4:2306-2315, which is incorporated herein by reference, demonstrate the cloning of the A. awamori glucoamylase gene, and Boel, et al., EMBO J. (1984) 3:1581-1585 describe two intervening sequences in the A. niger glucoamylase gene. The nucleotide sequence of a 2.7kb genomic fragment containing the N. crassa am (NADP-specific glutamate dehydrogenase) gene is reported by Kinnaird and Fincham, Gene (1983) 26:253-260 and an unstable mutant gene at the am locus is reported by Rambosek and Kinsey, Gene (1984) 27:101-107. Hughes, et al., Proc. Natl. Acad. Sci. U.S.A. (1983) 80:1053-1057 describe difficulties associated with producing a functional neomycin phosphotransferase enzyme (obtained from Tn903) in N. crassa.

## SUMMARY OF THE INVENTION

Hybrid DNA constructs are provided comprising an expression cassette for secretion of peptides comprising in the direction of transcription, a transcriptional and translational initiation region functional in a filamentous fungus, at least a portion of a signal leader or secretory leader sequence and, optionally, a processing signal, one or more restriction sites for insertion of an open reading frame coding for a protein of interest or the open reading frame, and a transcriptional termination region, which may or may not include translational termination signals. The construct is introduced into the filamentous fungal host under conditions where the construct is integrated into the host. The host may then be grown and the secreted product isolated and purified.

## DESCRIPTION OF THE SPECIFIC EMBODIMENTS

Novel DNA constructs are provided for use in filamentous fungal hosts for the expression and secretion of proteins of interest. The constructs are stably maintained in the filamentous fungal host, either by employing a stable extrachromosomal element or by providing for integration, particularly multiple integration, of the constructs into the filamentous fungal host cell genome. The construct will usually be produced by combining the various elements stepwise and cloning the product after each combination in an appropriate prokaryotic vector.

The expression constructs of interest will have in the order of transcription, 5′-3′ of the coding or sense strand, a transcriptional and translational initiation region, a signal leader, which may be all or part of a naturally occurring signal leader functional in filamentous fungi, and optionally a processing signal, one or more restriction sites for insertion of an open reading frame encoding a protein of interest or the protein of interest (other than the open reading frame naturally associated with the signal leader) and a transcriptional termination region functional in filamentous fungi, optionally including at its 5′ end translational stop signals.

The transcriptional initiation region may be derived from a wide variety of sources, endogenous or exogenous to the expression host. Thus, any translational and transcriptional initiation region may be employed which is functional in the fungal host, although initiation regions will be preferred which are endogenous to the same or different filamentous fungus as the host. The initiation regions may be constitutive or inducible so that continuous or regulated transcription is obtained. The transcriptional initiation region may be obtained from viruses, filamentous fungi, or other source, as appropriate. Illustrative transcriptional initiation regions include those regions associated with the glucoamylase gene, the glutamate dehydrogenase gene, the beta-tubulin gene, the glycolytic enzyme genes, such as glyceraldehyde-3-phosphate dehydrogenase, and the like. These genes may be obtained from various species of such genera as Aspergillus, Neurospora, Fusarium, etc. Species among this genera include N. crassa, A. niger, A. nidulans, A. awamori, P. chrysogenum, F. solani pisi, etc.

For inducible expression, regulatory regions may be obtained from a wide variety of genes, such as heat shock genes, glycolytic enzyme genes, glucoamylase genes, etc. The regulatory region may be used in conjunction with the RNA polymerase binding region or may be joined to a different RNA polymerase binding region to provide for a chimeric transcriptional initiation region. The regulatory regions may be obtained from any source, which provides for functional regulation in the filamentous fungal host.

The transcriptional initiation region will generally be from about 0.1 to 2kbp, usually 0.2 to 1kbp.

The signal leader will be comprised of a region which provides for transfer of the nascent protein into the lumen of the endoplasmic reticulum. All or a portion of the naturally occurring signal leader may be employed, usually at least about 60%, more usually at least about 80% of the coding sequence will be present to provide for the secretory function. The processing signal may or may not be present depending upon the purpose of the product, the need to remove the signal leader from the protein product or the availability of an alternative processing signal, which allows for subsequent processing in vivo or in vitro. Processing signals may involve a simple di-or polypeptide of basic amino acids which are recognized by proteases or may involve signals recognized by specific peptidases. In addition, the processing signal may involve spacers, which are removed by peptidases after cleavage of the peptide bond removing the signal leader. For subsequent cleavage, a methionine may be introduced at the cleavage site, where cyanogen bromide may serve to cleave at that site.

In order to provide for convenient introduction of the gene of interest in reading frame with the signal leader and, as appropriate, processing signal, the nucleotides coding for the region proximal to the 3′ terminus of the signal leader or coding for the processing signal or cleavage site may be conservatively mutated so as to provide for a convenient restriction site. Alternatively, restriction sites may be introduced subsequent to the signal leader or cleavage site for insertion of the open reading frame of interest, where the presence of superfluous amino acids at the N-terminus of the peptide of interest will not have an adverse effect on the use of the protein product. Alternatively, adaptors may be employed which provide for recreating portions of the signal leader, processing signal and/or open reading frame, so as to provide for the open reading frame being in proper reading frame with the signal leader and, when appropriate, processing signal. The open reading frame will normally terminate in one or more stop codons for proper processing of the C-terminus of the protein product.

The termination region may be any convenient region from any source which is functional in the filamentous fungal host. The termination region may be associated with the same or different gene as the transcriptional initiation region. Usually, the transcriptional initiation region will be from about 100 to 500bp, although there need be no upper limit.

The expression construct including the gene may be used by itself for transformation, particularly where integration is desired, or may be joined to other DNA sequences for a variety of purposes.

One DNA sequence with which it may be joined is a DNA sequence homologous with a DNA sequence of the host cell. This DNA sequence will usually be at least about 50bp, more usually at least about 100bp, and may be 2000bp or more. The sequence may include a gene which complements an auxotrophic host, so as to provide prototrophy to the host and selection of organisms which have been transformed with the desired DNA. Alternatively, one can use a selectable dominant marker, which imparts biocide resistance to the host. Particularly, antibiotic resistance markers find use, such as resistance to kanamycin, neomycin or G418. One gene which is capable of imparting this resistance is the neomycin phosophotransferase II gene, which is found on Tn5. Other markers of particular interest include resistance to the fungicide Benomyl. The dominant marker gene will be provided as an expression cassette having the transcriptional and translational initiation and termination signals appropriate to expression of the dominant selectable marker in the filamentous fungal host.

Since the various constructs will normally be prepared in a prokaryotic vector, particularly a vector capable of replication in E. coli, a prokaryotic replication system may be joined to the expression construct. Various replication systems are well known, commonly pBR322, pACYC184 and pUC(series) have found application. In addition, the prokaryotic replication system will also usually include a marker which allows for selection in prokaryotes, particularly a complementation marker for biocide resistance, particularly antibiotic resistance or fungicide resistance.

The expression construct, by itself or in combination with additional DNA, may then be used for transformation of filamentous fungi. Transformation can be achieved by preparing spheroplasts from mycelia, isolating the spheroplasts free of cellular debris, and transforming by combining the spheroplasts with the transforming DNA in an appropriately buffered medium and allowing the mixture to incubate. The spheroplasts may then be isolated, suspended in an appropriate nutrient medium for regeneration of cell walls and, where a marker is present, combined with a selective medium for selection of transformants. The transformants may then be isolated amplified and used for expression of the desired protein.

A wide variety of proteins are of interest and may be prepared in accordance with the subject invention. For the most part, the proteins will be at least about 1000 molecular weight, more usually at least about 2000 molecular weight, and generally less than about 1 million molecular weight, usually less than about 600,000 molecular weight. The primary proteins of interest will generally range from about 30,000 to 400,000 molecular weight, more usually from about 30,000 to 250,000 molecular weight. The proteins of interest may be derived from prokaryotic or eukaryotic sources. Of particular interest are mammalian proteins, more particularly human proteins.

Illustrative proteins of interest include enzymes, such as glucoamylase, blood proteins involved in the clotting or clot-dissolving cascades, such as thrombin, Factor VIIIC, Factor IX, Factor XI and Factor XII, tissue plasminogen activator, plasminogen, urokinase, streptokinase, serum albumin, etc.; hormones, such as insulin, somatomedin, somatostatin; lymphokines, such as the interleukins, -1, -2, and -3; growth factors such as platelet-derived growth factor, tumor necrosis factor, epidermal growth factor, skeletal growth factor, alpha-transforming growth factor, etc.; surface membrane proteins, such as the major histocompatibility complex proteins, growth factor receptors, outer membrane proteins; proteins from pathogens, such as viruses, e.g. hepatitis, LAV, feline leukemia virus, herpes virus; Chlamydia, Neisseria gonorrhea, Enterobacertiaceae; structural proteins, such as collagen, keratin, and the like; and synthetic proteins of interest.

The filamentous fungi which find employment may be from Neurospora, Aspergillus, Fusarium, Penicillium or the like. Species among this genera include N. crassa, A. niger, A. nidulans , A. awamori, P. chrysogenum, F. solani pisi, etc.

The transformed hosts may be selected for by the copy number of the expression construct, the production of product from the expression construct, or other factors of interest for commercial exploitation. Thus, the transformants will be screened as to the properties of interest which allow for efficient economical expression and secretion of the desired protein product. Once the transformants have been selected for use, they may then be used in conventional ways for the production of the desired product.

In some instances, it may be desirable to combine the expression construct with an amplifiable gene, such as DHFR, TK, or a metallothionein gene. By stressing the transformant cells, the number of integrated copies may be greatly amplified, so as to further increase the amount of expression of the desired product. In addition, it may be desirable to include in the construct or in a separate construct which is cotransformed, an expression product which provides for a peptidase for cleavage of the processing signal, where such processing signal is present. In this instance, one may avoid overwhelming the naturally occurring processing system of the host, so as to permit an increased level of secretion.

The following examples are offered by way of illustration and not by way of limitation.

EXPERIMENTAL

For the following experiments, the DNA manipulations were carried out in accordance with well-known procedures, such as described in Maniatis, T., etal., "Molecular Cloning, A Laboratory Manual," Cold Spring Harbor Laboratory, U.S.A. (1982), which is incorporated herein by reference.

All enzymes and plasmids, unless otherwise stated, are available from various commercial sources, such as New England Biolabs, Beverly, Massachusetts; Collaborative Research, Waltham, Massachusetts; Miles Laboratories, Elkhart, Indiana; Boehringer Biochemicals, Inc., Indianapolis, Indiana; and Bethesda Research Laboratories, Rockville, Maryland. Buffers and reaction conditions for restriction enzyme digestion were used according to recommendations supplied by the manufacturer for each material, unless otherwise indicated. All cited publications and patents are specifically incorporated herein by reference.

The following stock solutions were utilized:

1. KMP = 0.7M KCl/0.8M mannitol/20mM $KPO_4$, pH 6.3.

2. KMPC = KMP plus 50mM $CaCl_2$ (Ca added just before use).

3. Heparin = KMPC with 5mg heparin/ml, made fresh before each use.

4. PPC = 40% polyethylene glycol (Sigma 3500)/20mM $KPO_4$ (pH 6.3)/50mM $CaCl_2$.

5. Regeneration agar

20 x Mineral salts 50ml

Tryptone 5g

Yeast extract 5g

Glucose 10g

Mannitol 236.86g

Adjusted to one liter with water, heated when necessary to dissolve the mannitol. To each 125ml media bottle, 1.5 grams agar and 50ml of above solution were added.

6. Overlay agar. 1% peptone and 0.7% agar, made in 50ml aliquots.

7. 20 x Mineral salts

$NaNO_3$ (sodium nitrate) 120g

KCl 10.4

$MgSO_4 \cdot 7H_2O$ 10.4g

$KH_2PO_4$ 30.4

Vogel's trace elements 2.0ml

Deionized water to one liter

Each salt should be dissolved completely before adding the next. Stored at room temperature with 2ml of chloroform as preservative. Used at 50ml per liter.

8. Vogel's trace element:

Deionized water 95ml

Citric acid (not sodium salt) 5g

$ZnSO_4 \cdot 7H_2O$ 5g

$Fe(NH_4)_2 (SO_4)_2 \cdot 6H_2O$ 1g

$CuSO_4$ 0.25g

$MnSO_4 \cdot H_2O$ 0.05g

$H_3BO_3$ 0.05g

$Na_2MoO_4 \cdot 2H_2O$ 0.05g

Each ingredient is dissolved before adding the next, stored at about 4°C, and used at 0.1ml per liter of medium.

9. Complete Medium

20 x Mineral salts 50ml

Tryptone 5g

Yeast extract 5g

Glucose 10g

Water qv 1L

## Proinsulin Trimer Secreted Using Glucoamylase Leader, Promoter-Terminator

### Plasmid Construction

1. pNEO was purchased from Pharmacia P-L Biochemicals (Cat #27-4924).

2. pPL1: The 2kb EcoRI-BamHI fragment of pJR2 (containing the 5' region and coding sequences plus a portion of 3' non-coding sequences of the N. crassa am gene; nucleotides 1-2034, numbered according to J.H. Kinnaird and J.R.S. Fincham, "The complete nucleotide sequene of the Neurospora crassa am (NADP-specific glutamate deydrogenase) gene," Gene 26:253-260 (1983) and obtained from J. Kinsey, University of Kansas Medical Center. Kansas City), was isolated and cloned between the EcoRI and BamHI sites of pBR322.

3. pPL1-R: pPL1 was cut with ClaI (at nucleotide 283, numbered according to Kinnaird and Fincham, supra) and the cohesive ends were made flush by incubation with reverse transcriptase and a mixture of the 4-deoxyribonucleotide triphosphates. Then the plasmid was incubated with synthetic phosphorylated EcoRI linkers (GGAATTCC), the material cut with EcoRI, and the plasmid ligated.

4. pUTX27: pBR322 was cut with EcoRI, filled with the Klenow fragment of DNA polymerase, and reclosed. This removed the EcoRI site.

5. pEV1: pUTX27 was cut with BamHI and phosphatased. This was ligated to the ≈300bp BamHI-EcoRI fragment of pPL1-R (which contains the promoter and transcription start site of the am gene) and the ≈600bp EcoRI-BamHI fragment from pJR2 (nucleotides 2030-2644 numbered according to Kinnaird and Fincham, supra) which contains the sequences surrounding the polyA addition site of the am gene. The resulting plasmid contains a unique EcoRI site between the transcription start and stop sites of the am gene.

6. pRRNEO: The BglII-Ava I fragment of pNEO (nucleotides 1516-2520 numbered according to E. Beck, G. Ludwig, E. A. Auserwald, B. Reiss and H. Schalle, "Nucleotide sequence and exact location of the neomycin phosphotransferase gene from transposon Tn5," Gene 19:327-336 (1982); available from P-L Biochemicals) containing the coding sequences for the neomycin phosphotransferase II gene, was excised, ligated to EcoRI linkers (8mer) and cloned into the EcoRI site of pUC8.

7. pEV1neo(+): The 1kb EcoRI fragment from pRRNEO was cloned into the EcoRI site of pEVI. The orientation of the plasmid is am 5'-NPTII(5-3)-am 3'. This plasmid contains an out-of-frame ATG at 16 bases 5' to the major translation start of the NPTII gene.

8. pAF1: pUC8 with a filled-in EcoRI site.

9. pEV2neo: The BamHI fragment of PEV1neo was cloned into the BamHI site of plasmid pAF1.

10. NEObal-3: pNEQ was opened with BglII and resected with Bal31 nuclease. EcoRI linkers (8mer) were added and the plasmid was closed. The specific plasmid contains an Eco RI linker attached immediately adjacent to nucleotide 1543 (numbered according to Beck, et al., supra) in the 5' region of the NPSII gene. This removes the out-of-frame ATG.

11. p42-6-1: pNEObal-3 was opened with AvaI, filled in with the Klenow fragment of DNA polymerase, and EcoRI linkers (8mer) were added at this site. The plasmid was cut with Eco RI and the resulting ≈1kb EcoRI fragment (which contains the coding sequence for NPTII) was cloned into the EcoRI site of pUC8.

12. pEV2neobal: the EcoRI fragment of p42-6-1 was introduced to replace the EcoRI fragment of pEV2neo. The orientation is am 5'-NPTII (5'-3')-am 3'.

13. pEV3neobal: The BamHI fragment of pEV2neobal was introduced into the BamHI site of pUC18.

14. pEV3neobalΦXho: The XhoI site in the 3' am sequences of pEV3neobal was filled.

15. pGAN: A 10kb HindIII-XbaI fragment from AG111 (containing the glucoamylase gene) was cloned between the HindIII and XbaI sites of pEV3neobalΦXho).

### Isolation of the A. Niger Glucoamylase Gene

A Sau2A-partial library of A. niger DNA (from strain ATCC 1015) was constructed in lambda phage EMBL4 (A. M. Frischauf, H. Lehrach, A. Poustra and N. Murray, "Lambda Replacement Vectors Carrying Polylinker Sequences", J. Mol. Biol. (1983) 170:827-842, available from Promega). The glucoamylase gene was isolated from this library by screening plaques with a 26mer probe (GCGGACGGTGCTTGGGTGTCGGGCGC). The sequence of the probe was taken from the published glucoamylase sequence: J.H. Nunberg, J.R. Meade, C. Cole, F.C. Lawyer, P. McCabe, V. Schweichart, R. Tal, V.P. Wittman, J.E. Flatgaard, and M.A. Innis, "Molecular Cloning and Characterization of the Glucoamylase Gene of Aspergillus awamori," Molecular and Cellular Biology (1984) 4:2306-2315. The phage used in these experiments was termed lambda AG111. This phage contains the complete glucoamylase gene as part of a 19kb insert. The restriction map agrees with the published information about the gene from A. nigerstrain BU-1 and A. awamori (NRRL 3112): E. Boel, M.T. Hansen, I. Hoegh and N.P. Fiil, "Two different types of intervening sequences in the flucoamylase gene from Aspergillus niger," The EMBO Journal (1984) 3:1581-1585; and J.H. Nunberg, J.R. Meade, G. Cole, F.C. Lawyer, P. McCabe, V. Schweichart, R. Tal, V.P. Wittman, J.E. Flatgaard, and M.A. Innis, "Molecular Cloning and Characterization of the Glucomylase Gene of Aspergillus awamori," Molecular and Cellular Biology 1984) 4:2306-2315. (All of the above references are specifically incorporated herein by reference.)

plac239/3PI: The proinsulin trimer is in EcoRI-BamHI insert. This plasmid is fully described in Shen, S-H., Proc. Natl. Acad. Sci. U.S.A. (1984) 81:4627-4631. The plasmid was supplied by Dr. Shi-Hsiang Shen of Connaught Research Institute, Willowdale, Ontario, Canada.

Glucoamylase Transcription Terminator

1. pKF17: pGAN was completely digested with HindIII partially cleaved with SalI. The cohesive termini were filled by incubation with the Klenow fragment of E. coli DNA polymerase I in the presence of the four dNTPs. The plasmid was reclosed by blunt end ligation and transformed into E. coli. The desired plasmid (identified by restriction mapping) contained a deletion starting 5' to the SalI site at the 3' end of the coding region of the glucoamylase gene (i.e., the middle of the SalI site of the A. niger DNA insert) and extending to the HindIII site which bordered the original A. niger DNA insert. (The SLI site which borders the deletion site is destroyed, but the HindIII site which borders the other end of the deletion is reformed upon ligation to the filled-in SalI site.)

2. pKF27: pKF17 was cleaved at the unique HindIII site, the cohesive ends filled in, and the synthetic BglII linkers (CAGATCTG) were ligated to the ends. The cohesive ends of the linkers were revealed by cleavage with BglII and the plasmid was religated. (The HindIII site is lost in this manipulation.)

Glucoamylase Promoter Plus Leader Sequence

1. pKF20: pGAN was cleaved with BssHII, which cleaves the plasmid at the sequence encoding the junction between the leader peptide and the mature glucoamylase protein (as well as one time in the region encoding the mature enzyme and one further time in the neomycin phosphotransferase (nptII) gene) and the plasmid religated and transformed into E. coli. The desired plasmid had a deletion which extended from the BssHII site at the leader peptide to the BssHII site in the neomycin phosphotransferase gene.

2. pKF25: pKF20 was cleaved at the unique BssHII site, the cohesive termini filled in, the synthetic BglII lilnkers (CAGATCTG) added. (Addition of the BglII linkers to the properly filled BssHII site results in the reformation of the BssHII site.)

Joining Terminator to Promoter and Leader

pKF29-9: The large (4.6kb) XbaI-HindIII fragment of the pEV3neobal (containing the bacterial origin of replication, bacterial selectable marker as well as fungal selectable marker), was ligated to the 3.7kb XbaI-Bgl II fragment of pKF27 (containing the terminator fragment) and the 4.3kb HindIII-BglII fragment of pKF25 (containing the promotor and leader sequences). All fragments were gel purified prior to the ligation. The proper plasmid was determined by restriction mapping.

Note that this plasmid has a unique BglII site located between the leader peptide encoding sequence and the transcription terminator sequence. The appropriate translational reading frame at this sequence is:

```
      BssHII  BglII
      AAG CGC GCA GAT CTG AGCTCGAC....
      Lys Arg/Ala Asp Leu .... (pKF29-9 sequence)
      Lys Arg/Ala Thr Leu ...: (normal glucoamylase
                                    sequence)
(The "/" denotes the normal cleavage site)
```

## Adjusting translational reading frame of proinsulin

pKF31-16: Plasmid plac239/3PI was cleaved with EcoRI (at the 5′ end of the proinsulin sequence), the cohesive termini filled and synthetic BgIII site ligated adjacent to an EcoRI site (the correct filling and ligation were inferred by the reformation of the EcoRI site).

## Final construction

pKF34-16: The proinsulin trimer with the correction translation reading frame was excised from pKF31-16 by cleavage withBgIII plus BamHI and inserted into the BgIII site of pKF29-9. The plasmids were screened by restriction mapping for one which contained the proinsulin trimer inserted in the proper orientation.

The plasmid is essentially pGAN with the glucoamylase coding region replaced with the coding sequence for the proinsulin trimer.

## Secretion of Proinsulin

Plasmid pKF34-16 was introduced in A. niger strain ATCC 1015 by transformation as follows:

A. niger were grown on agar slants (consisting of 2% glucose, 1% peptone, 2% agar) for 8-12 days at 30°C. Conidia were suspended in a solution of 0.005% Nonidet P-40. The conidial suspension was shaken in a tube with glass beads to break up the conidial chains, and then used to inoculate a flask of complete medium (solution 9) (100 ml in a 500ml Erlenmeyer flask) to a concentration of 5-10x$10^5$ conidia/ml for incubation at 30°C, 200rpm, 24hr. Thereafter, mycelia were harvested by filtering onto a double layer of cheesecloth and washed with KMP (solution 1), with the excess liquid squeezed out. The mycelia were added to a sterile 500ml flask with 50ml KMP, 500mg Novozym 234 (Novo Laboratories, Wilton, CT 06897) and incubated at 30°C at 70rpm overnight. To separate spheroplasts from mycelial cell debris, the solution was filtered through a double layer of cheesecloth and glass wool into conical centrifuge tubes, then the filtrate was centrifuged at 1800rpm for 15min in a bench top centrifuge. The supernatant was discarded and the pellet of spheroplasts was washed two times (in order to remove the Novozym) by resuspension in KMP and recentrifugation.

For transformation, 5-50 μg DNA in 20μl 0.01M Tris, 0.001M EDTA (pH 8.0) was added to 6.2μl heparin solution, and incubated at room temperature for about 10 minutes. The pellet of spheroplasts from the recentrifugation was resuspended in KMPC to 5x$10^7$/ml, and 200μl of the spheroplast suspension was added to the DNA with 50μl PPC, mixed gently and incubated on ice for 30 minutes. Then, 1ml PPC was added and the transformation mix added to 50ml of regeneration agar at 50°C containing 0.5mg G418/ml. (The G418, as 125μl of 200mg G418/ml stock solution was added just before adding the spheroplasts.) This mixture was swirled and poured into 4 Petri dishes, cooled quickly, then incubated at 30°C for 4 hours. The plates were then overlayed with overlay agar (containing about 3mg G418/ml) with the number of mls equal to the weight of the regeneration agar mixture added to the dish. The dishes were incubated at 30°C, and transformants appeared in 3 to 7 days.

Transformants were detected by selecting for resistance to G418. One transformant, termed ABM47, was taken for further analysis. The strain was a stable transformant and contained multiple copies of pKF34-16 inserted in tandem into the chromosomal DNA of the organism. The site of integration was not absolutely determined, but Southern hybridization revealed that the plasmid was not integrated at the normal glucoamylase locus. No alterations were formed in the restriction map of the normal glucoamylase locus.

Strain ABM47 was grown at 35°C at 150rpm in a volume of 25ml in a 250ml Erlenmeyer flask for periods of up to seven days. The media employed was complete media (Medium #9) containing either glucose (2-10%) or soluble starch (2-10%) as a carbon source. Samples were taken from the transformant at times ranging from 17 hours to 5 days of cultivation and RNA was prepared and subjected to Northern blot analysis. All samples contained RNA of the expected size, which cross hybridized with a labeled proinsulin DNA probe.

Strain ABM47 was grown as above (on 10% glucose as a carbon source) for 5 days. The culture fluid was collected by filtration and concentrated by precipitation with 75% acetone. The concentrated culture fluid was resuspended in SDS containing buffer and subjected to SDS-polacrylamide gel electrophoresis. Adjacent lanes on the gel contained molecular weight standards. Following electrophoresis, the gel was stained with Coomassie Brilliant Blue. A band of protein (approximate Mr = 30,000, the size expected for the proinsulin trimer) was observed in the transformed strain ABM47, but absent in the control untransformed strain A. niger ATCC 1015. The amount of the putative proinsulin trimer was approximately 1mg/liter of culture filtrate.

Culture fluid from strain ABM47 is subjected to gel electrophoresis on an SDS polyacrylamide gel and the protein is transferred to nitrocellulose by the procedure of Towbin, et al. (Proc. Natl. Acad. Sci. U.S.A. - (1979) 76:4350-4354). The resulting gel blot is probed with guinea pig antiserum directed against porcine insulin (Miles). The site of antibody reaction is detected with horse radish peroxidase conjugated anti-guinea pig IgG (Miles). The Mr = 30,000 protein is expected to be the only material which crossreacts with the antibody.

Dot blot analysis of culture fluid with the above antibody, using porcine insulin as a standard (and correcting for differential cross relativity of porcine insulin and human proinsulin) reveals at least 1 mg/liter of proinsulin trimer produced after 5 days of cultivation using 10% glucose as a carbon source.

The proinsulin may be purified from the culture fluid by precipitation of the culture fluid with 75% acetone, resuspending the pellet in a small volume of buffer, and separating the proinsulin from the other culture fluid proteins (primarily glucoamylase) by gel filtration on a column of Sephadex G75.

Amino acid sequence analysis of the purified proinsulin by a gas phase sequenator reveals the presence of the sequence Ala Asp Leu Asn Ser Met Phe... as expected for proper cleavage of the recombinant protein after the leader sequence. The junction hexapeptide prior to the authentic first amino acid of proinsulin (phe) may be removed by cleavage of the peptide with cyanogen bromide according to the conditions described by Shen etal., supra.

Proinsulin Trimer Secreted Using Glucoamylase Promoter-Terminator and Cutinase Leader

Plasmid Construction

1. Starting plasmids.

pEV3neobal: Source of selectable marker (see above).

p42-2-1: A 3.4kb EcoRI fragment (containing the complete glucoamylase gene) obtained from lambda AG111 was cloned into the EcoRI site of pUC8.

p50-1-16: p42-2-1 was cut with BssHII and resected with Bal31 nuclease. XhoI linkers (CCTCGAGG) were added and the plasmid was closed. The specific plasmid contains an XhoI linker attached immediately adjacent to nucleotide -37 (numbered relative to the normal translation start of the glucoamylase gene as + 1).

p56-4-7: The EcoRI fragment of p50-1-16 (containing the Bal31 deleted glucoamylase gene) was moved to the EcoRI site of pUC8ΔSal.

C31: cutinase cDNA cloned in pUC9. This plasmid is fully described in Soliday, et al., Proc. Natl. Acad. Sci. U.S.A. (1984) 81:3939-3943.

2. Isolation of the sequence encoding cutinase leader peptide and fusion to the glucoamylase promoter.

1. p56-4-7-B: p56-4-7 is cleaved with HindIII (within the pUC8 polylinker sequence located 3' to the insert), the cohesive ends are filled and BglII linkers (CAGATCTG) are ligated on. The DNA is cleaved with BglII to reveal the cohesive termini and the plasmid is recircularized. (This plasmid will ultimately link the glucoamylase promoter to the cutinase leader.)

2. pUC18HX: pUC18 is cleaved with HindIII, the cohesive ends are filled and XhoI linkers (CCTCGAGG) are ligated on. The DNA is cleaved with XhoI to reveal the cohesive termini and the plasmid is recircularized.

3. pCUT: pC31 is cleaved with NlaIII and the 766bp fragment (starting on the mRNA identical strand at nucleotide + 4 (numbered relative to the translational start as + 1) is purified and cloned into the SphI site of pUC18X. A plasmid is selected oriented so that the 5' end of the coding region is closer to the XhoI site of the plasmid.

4. pCUT-B: pCUT is linearized by partial digestion with the enzyme AluI and BglII linkers (CTAGATCTAG) are attached. The DNA is digested with BglII to reveal the cohesive termini and the plasmid is recircularized. The proper plasmid (selected by restriction mapping and DNA sequencing) has the BglII linkers inserted at the AluI site which cleaves between the sequence encoding amino acid residues 30 and 31 of cutinase.

5. pAGC1: i. pCUT-B is cleaved with BglII and Xho I and the 106bp fragment containing the sequence encoding the cutinase leader is purified.

ii. p56-4-7-B is cleaved with BglII plus XhoI and the large fragment (containing the glucoamylase promotor in the pUC8 backbone) is purified.

iii. The purified fragments i + ii are ligated together. The correct junction of these fragments places the glucoamylase promotor 5′ to the cutinase leader sequence.

6. pUC8Bgl: pUC8 is cleaved with EcoRI, the cohesive ends are filled, BglII linkers (CAAGATCTTC) are added, destroying the EcoRI site, the DNA is cleaved with BglII and the plasmid is recirculated.

7. pAG4: The Hind III-BglII insert of pKF25 (which contains the glucoamylase promotor and leader sequences) is cloned between the Hind III and BglII sites of pUC8Bgl.

8. pAGC2: pAG4 is completely digested with BglII (at the 3′ end of the glucoamylase leader) and partially with EcoRI (to cleave at the 5′ end of the glucoamylase promotor). The purified EcoRI-BglII fragment of pAGC1 is inserted into this plasmid. The result of this manipulation is to replace the glucoamylase promoter-leader with the glucoamylase promoter-cutinase leader.

3. Joining the glucoamylase terminator to the composite promoter-leader. (This construction is parallel to pKF29-9 described above.)

pAGC3: The large (4.6kb) XbaI-HindIII fragment of pEV3neobal (containing the bacterial origin or replication, bacterial selectable marker as well as fungal selectable marker), was ligated to the 3.7kb XbaI-BglII fragment of pkF27 (containing the terminator fragment) and the 4.3 kb HindIII-Bgl II fragment of pAGC2 (containing the promotor and leader sequences). All fragments were gel purified prior to the ligation. The proper plasmid was determined by restriction mapping.

Note that this plasmid has a unique BglII site located between the leader peptide encoding sequence and the transcription terminator sequence. The appropriate translational reading frame at this sequence is:

```
                    BglII
        GCG CGC CAG CTA GAT CTG AGCTCGAC
        Ala Arg Gln Leu/Asp      (pAGC3 sequence)
        Ala Arg Gln Leu/Gly      (normal cutinase sequence)
    (The "/" designates the normal cleavage site)
```

The translational reading frame at the BglII site is the same as for pKF29-9.

4. Final Construction. (This is a parallel construction to the prior construction using the glucoamylase leader.)

pAGC-3PI: The proinsulin trimer with the corrected translation reading frame was excised from pKF31-16 (see above) by cleavage with BglII plus BamHI and inserted into the BglII site of pAGC3. The plasmids were screened, by restriction mapping, for one which contained the proinsulin trimer inserted in the proper orientation.

This plasmid is essentially pGAN with the glucoamylase coding region replaced with the cutinase leader fused to the coding sequence for the proinsulin trimer.

Secretion of Proinsulin

Plasmid pAGC/3PI is introduced into A. niger strain ATCC 1015 by transformation and insulin production examined essentially as described above.

Rat Pancreatic Ribonuclease Secreted Using Glucoamylase Leader, Promoter and Terminator or Using Glucoamylase Promoter Terminator and Cutinase Leader

Plasmid construction

1. Starting plasmids.

p35-C-14: A beta tubulin gene from a dominant Benomyl resistant mutant of A. niger was cloned by cross-hybridization to a cloned beta tubulin gene of A. nidulans (provided by Cr. N.R. Morris, Rutgers University). A 5kb (EcoRI fragment containing a Benomyl resistance (BenR) gene was inserted into the EcoRI site of pUC8.

pcXP6-105: Rat pancreatic ribonuclease cDNA clone. The clone is described in MacDonald, R.J., J. Biol. Chem. (1982) 257 :14582-14585, and was provided by Dr. Raymond J. MacDonald, Department of Biochemistry, University of Texas Health Science Center, Dallas, TX.

2. Isolation of RNase coding sequence and adjustment of translational reading frame for fusion.

p62-C-1: The EcoRI-PstI fragment of pcXP-105 (containing the RNase coding region) was cloned into pUC18.

p81-C-13: Plasmid p62-C-1 was cleaved with EcoRI, the cohesive ends filled, and HindIII linkers attached. The DNA was digested with Hind III and the HindIII bounded fragment containing the RNase coding region was coned in the HindIII site of pUC8. The correct plasmid was oriented with the 5' end of the coding region towards the EcoRI site of the pUC8 polylinker.

pKF42: The SalI-SphI fragment of p81-C-13 was cloned between the SalI and SphI sits of pUC18.

pKF43: pKF42 was cleaved with XbaI (complete digest) and HinfI (partial digest). The cohesive termini were filled and the correct partially deleted plasmid (from the XbaI site to the second HinfI site in the RNase sequence) was gel purified and recircularized. The correct filling of the cohesive ends was confirmed by digestability of the plasmid with both XbaI and Hinf I. The sequence around the deletion was:

```
  BamHI    XbaI   HinfI
  G GAT CCT CTA GAA TCA TCG      (sequence of pKF43)
  Ala Asp Pro Leu Glu Ser Ser    (sequence of RNase
                                  produced when BamHI site
                                  of pKF43 is fused to the
                                  glucoamylase leader
                                  region of pKF29-9 at the
                                  BglII site.)

  Gly Glu Ser Arg Glu Ser Ser    (normal sequence of
                                  RNase)
```

pKF44: pKF43 with a Bgl II linker added at the HindIII site (3' to the RNase coding region).

### 3. Final constructions and expression .

The RNase coding sequences was excised from plasmid pKF44 by cleavage with BamHI plus BglII and the RNase encoding sequence isolated. This fragment was inserted into Bgl II cut pKF29-9 to provide plasmid p68-D-12. The construction allows for secretion of pancreatic ribonuclease driven by the glucoamylase promoter-leader. (Alternatively, secretion driven by the glucoamylase promoter-cutinase leader can be provided by inserting the RNase encoding sequence into BglII cut pAGC3.)

The details for plasmid p68-D-12 transformation of A. Niger are as follows. Plasmid p68-D-12 was introduced into A. niger strain ATCC1015 by cotransformation with plasmid p35-C-14 to provide a selectable marker. Plasmid p35-C-14 has a 5kb EcoRI fragment containing a Benomyl-resistant beta-tubulin gene from an A. Niger mutant inserted into the EcoRI site of pUC18. The transformation protocol was as described for the G418 selection, with the exception that transformants were detected by selecting for resistance to 1 mg/L Benomyl. The initial transformants were further screened for the presence of the glucoamylase-ribonuclease expression cassette by probing Southern blots of DNA prepared from the transformants with an RNase-specific probe. Transformants containing the expression construct were taken for further analysis. These strains were stable and contained multiple copies of the desired plasmid inserted in tandem into the chromosomal DNA of the organism. The site of integration was not absolutely determined, but Southern hybridization revealed that the plasmid was not inte grated at the normal glucoamylase locus. The restriction map of the normal glucoamylase locus was unaltered in these transformants.

Transformed organisms were grown at 35° at 150 rpm in a volume of 25 ml in a 250 ml Erlenmeyer flask for periods of up to seven days. The media employed was rich media containing 10% soluble starch as a carbon source. Samples were taken from the transformants at times ranging from 17 hours to 5 days of cultivation and RNA was prepared and subjected to Northern blot analysis. All samples contained RNA of the expected size, which cross hybridized with a labelled RNase-specific probe. No cross hybridization was observed for parallel samples prepared from untransformed control cultures. This result demonstrates that the expression construct is properly transcribed in the transformed organism.

Culture fluid taken from 3-day cultures of the transformants (grown as above) as well as control untransformed cultures was assayed for ribonuclease activity by standard procedures. The level of ribonuclease in the culture fluid obtained from the transformed culture was 20 mg/L (relative to the activity of a bovine pancreatic ribonuclease standard) while a value of 1 mg/L was obtained using culture fluid from the untransformed control culture.

Aliquots of the above culture fluid were diluted with SDS containing buffer and subjected to SDS-polyacrylamide gel electrophoresis. Adjacent lanes on the gel contained molecular weight standards as well as bovine pancreatic ribonuclease. Following electrophoresis the gel was stained with Coomassie Brilliant Blue. A band of protein having a mobility corresponding to a slightly larger Mr than the bovine pancreatic ribonuclease was observed in the transformed strains but was not seen in the control untransformed strain A. niger, ATCC 1015. Quantitation· of this stained protein (relative to a bovine pancreatic ribonuclease standard) revealed that its concentration was 20 mg/L of culture filtrate.

Further aliquots of the same culture fluids were fractionated on SDS-polyacrylamide gels which had been cast in the presence of 50 μg/ml of yeast RNA. The gel was subjected to in situ activity staining for ribonuclease activity after electrophoresis by soaking the gel in 1% Triton X-100, 0.01M Tris-HCl (pH 6.8) for one hour at room temperature, followed by 0.01M Tris-HCl (pH 6.8) for two hours at room temperature, followed by 0.01M Tris-HCl (pH 6.8) for one hour at 37°. The hydrolysis of the RNA substrate by the biologically active ribonuclease was revealed by soaking the gel in an aqueous solution of 2 μg/ml of ethidium bromide for 10 minutes and, following removal of the excess stain by soaking in 0.01M Tris-HCl for approximately 15 min, observing the localized lack of fluorescence when viewing the gel under ultraviolet light. This activity staining revealed the presence of a biologically active ribonuclease in the fractionated culture fluid of the transformed culture which migrated with the same mobility previously observed for the extra protein band in this culture fluid. No ribonuclease activity was detected by this activity staining for the fractionated culture fluid of the untransformed control culture.

It is evident from the above results, that filamentous fungi can be used for the efficient production of a wide variety of heterologous and homologous proteins. The resulting proteins are obtained in good yield and can be used for a variety of purposes, retaining their biological and physiological properties. The filamentous fungi are easy to grow and may be grown efficiently in accordance with known ways, so as to provide for economical production of proteins which may be used for treatment of mammals, with a substantial absence of materials detrimental to their use as injectables, such as enterotoxins, exotoxins, transforming DNA, and the like.

The plasmid p27 was deposited at the A.T.C.C. on May 30, 1986 and given Association No. 67123.

Although the foregoing invention has been described in some detail by way of illustration and example for purposes of clarity of understanding, it will be obvious that certain changes and modifications may be practiced within the scope of the appended claims.

## Claims

1. A DNA expression construct comprising a transcriptional and translational initiation region functional in a filamentous fungal cellular host, a functional signal leader comprising at least a fragment of at least 60 number percent of the codons of a signal leader of a structural gene coding for a protein secreted by a filamentous fungal cell, at least one restriction site for inserting a heterologous structural gene in proper reading frame with said signal leader or a structural gene other than the natural gene in proper reading frame with said signal leader, and a termination region functional in said filamentous cell.

2. A DNA expression construct according to Claim 1, wherein said signal leader is from a filamentous fungal glucoamylase gene or cutinase gene.

3. A DNA expression construct according to Claim 1, joined to a dominant selective marker gene capable of expression in said filamentous fungal cell.

4. A DNA expression construct comprising a glucoamylase transcriptional and translational initiation region functional in a filamentous fungal cellular host, a glucoamylase signal leader comprising at least a fragment of at least 60 number percent of the codons of said glucoamylase signal leader, at least one restriction site for inserting a heterologous structural gene in proper reading frame with said signal leader or a structural gene other than the glucoamylase gene in proper reading frame with said signal leader, and a termination region functional in said filamentous cell.

5. A DNA expression construct according to Claim 9, wherein a DNA sequence encoding a selective cleavage site separates said signal leader and said structural gene.

6. A DNA expression construct comprising a cutinase transcriptional and translational initiation region functional in a filamentous fungal cellular host, a cutinase signal leader comprising at least a fragement of at least 60 number percent of the codons of said cutinase signal leader, at least one restriction site for inserting a heterologous structural gene in proper rading frame with said signal leader, and a termination region functional in said filamentous fungal cellular host.

7. A filamentous fungal cell comprising a genome including a construct according to any one of the preceding claims, containing said structural gene, integrated into said genome at least once.

8. A filamentous fungal cell according to Claim 7, wherein said construct is joined to a dominant selective marker.

9. An <u>Aspergillus</u> fungal cell comprising a genome including a construct according to any one of claims 1 to 6, containing said structural gene, integrated into said genome at least once.

10. An <u>Aspergillus</u> cell according to Claim 9, wherein said structural gene is proinsulin gene.

| | DOCUMENTS CONSIDERED TO BE RELEVANT | | EP 87304477.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| P,A | WO - A1 - 86/06 097 (ALLELIX, INC.)<br>* Abstract *<br>-- | 1,9 | C 12 N 15/00<br>C 07 H 21/04<br>C 12 N 5/00<br>//(C 12 N 5/00<br>C 12 R 1:66) |
| P,A | EP - A2 - 0 184 438 (ALLELIX, INC.)<br>* Abstract *<br>-- | 1,9 | |
| A | GENE, vol. 26, 1983, Amsterdam<br>J.TILBURN et al. "Transformation by integration in Aspergillus nidulans"<br>pages 205-221<br>* Summary *<br>-- | 1,9 | |
| D,A | THE EMBO JOURNAL, vol. 4, no. 2, 1985, Oxford, GB<br>J.M.KELLY et al. "Transformation of Aspergillus niger by the amd S gene of Aspergillus nidulans"<br>pages 475-479<br>* Totality *<br>-- | 1 | TECHNICAL FIELDS SEARCHED (Int. Cl.4)<br><br>C 12 N<br>C 07 H |
| D,A | THE EMBO JOURNAL, vol. 3, no. 7, 1984, Oxford, GB<br>E.BOEL et al. "Two different types of intervening sequences in the glucoamylase gene from Aspergillus niger"<br>pages 1581-1585<br>* Totality *<br>-- | 1 | |

The present search report has been drawn up for all claims

| Place of search | Date of completion of the search | Examiner |
|---|---|---|
| VIENNA | 14-09-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

| | DOCUMENTS CONSIDERED TO BE RELEVANT | | EP 87304477.0 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | GENE, vol. 26, 1983, Amsterdam<br><br>J.H.KINNAIRD et al. "The complete nucleotide sequence of the Neurospora crassa am (NADP-specific glutamate dehydrogenase) gene" pages 253-260<br><br>* Totality *<br><br>---- | 1 | |
| | | | TECHNICAL FIELDS SEARCHED (Int. Cl.4) |

The present search report has been drawn up for all claims

| Place of search | Date of completion of the search | Examiner |
|---|---|---|
| VIENNA | 14-09-1987 | WOLF |